# EUROPEAN PATENT SPECIFICATION

(11) **EP 1 574 168 B1**
(45) Date of publication and mention of the grant of the patent: **23.04.2008**
(21) Application number: 04005675.6
(22) Date of filing: 10.03.2004
(51) Int. Cl.: A61B 17/00, A61M 25/01

(54) **System comprising a guide rod and first and second introducers**
System mit einem Führungsdraht und erster und zweiter Einführungsvorrichtung
Système comprenant une tige de guidage et premier et second introducteur

(43) Date of publication of application: 14.09.2005
(73) Proprietor: RADI MEDICAL SYSTEMS AB, 754 50 Uppsala (SE)
(72) Inventor: Egnelöf, Per, 754 40 UPPSALA (SE); Preinitz, Fredrik, 753 50 UPPSALA (SE); Tenerz, Lars, 756 46 UPPSALA (SE)
(74) Representative: Stavbom, Ellen Elisabet

(56) References cited:
- EP-A- 0 331 932
- EP-A- 0 397 173
- DE-C1- 4 319 033
- US-A- 5 478 326
- US-A- 5 695 111
- US-A1- 2003 195 560

## Description

### Field of the invention

The present invention relates to a guide rod, a first and second introducer in a system to be used generally in a method for replacing an introducer, which is inserted in a vessel, with another introducer, and more particularly to guide rod utilized for replacing an introducer with another introducer without the conventional use of a guide wire and a dilator.

### Background of the invention

In US 6,090,130, which is assigned to Kensey Nash Corporation, a system for sealing a percutaneous puncture in a blood vessel is disclosed. For the determination of the position of the blood vessel, the system comprises a blood vessel locator, which, in turn, comprises an introducer sheath and a dilator. The position of the blood vessel is determined by observing a flow of blood out from a hole in the proximal end of the introducer sheath. The hole in the proximal end of the introducer sheath is via a canal in fluid communication with a hole in the distal end of the vessel locator. US 6,090,130 discloses several different embodiments of how this canal and the distal hole can be arranged. A common requirement for these embodiments is, however, that the length of the dilator is adapted to the length of the introducer sheath.

US 6,090,130 describes also a typical, conventional, intravascular surgical procedure, in which an introducer sheath according to the patent can be used. It is said that in such a procedure, a cannula of an instrument, such as an angiographic needle, is inserted percutaneously through the skin into the artery, such as the femoral artery, at the situs for the instrument's insertion. The needle cannula is held in place and the flexible end of a mini-guidewire is then passed through the cannula into the artery to the desired length. Once the mini-guide is in place, the needle cannula is removed, leaving the guidewire in place. An introducer sheath and an arterial dilator are then passed over the guidewire, through the puncture or incision and into the artery. The guidewire and then the dilator are removed, leaving the introducer sheath in place. A catheter, or other intravascular instrument, is then inserted through the introducer sheath and threaded down the artery to the desired intravascular location.

In the above description of the intravascular surgical procedure, which is taken directly from US 6,090,130 and which is based on the so-called Seldinger technique, it is explicitly assumed (by reference numerals) that the introducer sheath being used during the intravascular procedure is the same introducer sheath as the introducer sheath into which a dilator is inserted when the intravascular operation has been completed and the sealing operation is to be performed. In other words, it is assumed that there is only one introducer sheath used during the whole medical operation, i.e. during both the actual intravascular procedure and the subsequent sealing operation. Although this could be the case, it is to the best of the present applicants' knowledge almost never actually the case. The reason for this is mainly that the needle cannula, introducer sheath, dilator, guide wire and catheter, and possibly some more instruments, come together in a separate set, which presumably is provided by another company than the company that provides the sealing system, i.e. in this particular example the Kensey Nash Corporation. Now, if the length of the special dilator, which is used to provide the canal in the vessel locator according to US 6,090,130, does not match the length of the introducer sheath which already is inserted in the artery, this introducer sheath has to be replaced with an introducer sheath having the suitable, known length. Since introducer sheaths come in a variety of dimensions, the first introducer, which is inserted in the artery, must usually be replaced, and a special replacement technique has been developed for this purpose. In one stage, this known replacement technique requires that a medically trained person apply manual compression pressure, and the technique requires further that two separate instruments, a guide wire and a dilator, are used.

US-A-5 478 326 (SHIU ET AL) discloses a flexible cannula having a central passage for threading the cannula over a guide wire. The cannula has a cross-sectional area continuously decreasing from a proximal (outer) end towards a distal (insertable) end, the proximal end carrying a seat integrally formed with the cannula and receiving a clamp screw by which the cannula optionally is secured to the guide wire running through the passage. This cannula is intended and designed to permit controlled closure of an arterial puncture by incrementally withdrawing the tapered cannula from the blood vessel. Typically, a cannula is formed with a lumen through which blood can flow, as is the case also with the cannula of this document. The cannula is thus not effective for blocking blood flow, nor is it intended or structurally suitable for shifting instruments insertable through the puncture. In US-A-5 478 326 a conventional guide wire is relied upon for introducing the cannula in a blood vessel.

### Summary of the invention

An object of the present invention is therefore to provide a system having a guide rod, a first and second introducer whereby the guide rod facilitates the replacement of the first introducer, whose distal end is inserted into a vessel and whose proximal end is outside the skin (or other tissue) of a patient, by the second introducer.

Another object of the invention is to provide a system having a guide rod which replaces the guide wire and the dilator used in the previously known replacement method.

These objects are achieved by a system according to independent claim 1. Preferred embodiments are set forth in the dependent claims.

According to the invention, a guide rod is first inserted into the first introducer, which is in place in the vessel. The first introducer is then removed, thereby leaving only the guide rod in place. Thereafter, the second introducer is passed over the guide rod and into the vessel, and in a last step the guide rod is removed.

To not damage the vessel, the guide rod according to the invention should preferably have a flexible distal portion with a blunt distal end, while the proximal portion, which has a tapered proximal end, should be stiffer to facilitate the threading of the second introducer.

### Brief description of the drawings

Figs. 1-5 illustrate the steps in a replacement procedure according to the prior art.
Figs. 6-10 illustrate the steps in a replacement procedure according to the present invention.
Fig. 11 shows the guide rod according to the present invention used in the replacement procedure.
Fig. 12 shows a tool for closing a puncture hole in a vessel.
Fig. 13 shows the tool of fig. 12 with a guide rod of the present invention inserted into a blood vessel.

### Detailed description of the preferred embodiments

Before describing the method made possible by the present invention, a brief description of the method previously utilized to replace a first introducer, whose distal end is inside a vessel and whose proximal end extends out of the skin of a patient, with a second introducer, will be provided. This well-known method may be used, as mentioned above, to replace the first introducer with a second introducer having a length that matches the length of a dilator, which is designed in such a way that the combination of the dilator and the second introducer can be used as a vessel locator. The method could, however, be used whenever one wants to change introducer, for instance when a new introducer having a larger diameter is needed.

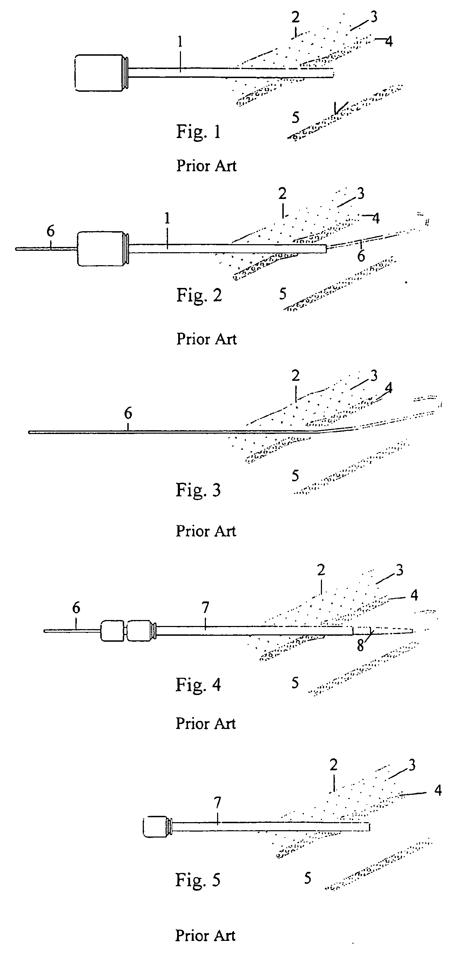

Fig. 1 illustrates a situation in which a first introducer 1 has been inserted through a puncture hole, which extends from the skin 2 of a patient, through tissue 3 and through the wall 4 of a blood vessel 5, such that the distal end of the introducer 1 is inside the blood vessel 5 while the proximal end of the introducer 1 is outside the skin 2. The situation illustrated in Fig. 1 represents a starting point for the conventional method to be described below in conjunction with Fig. 2 to Fig. 5.

In Fig. 2, the first step of the conventional method is shown. In this first step, a guide wire 6 is introduced into the introducer 1 until the distal end of the guide wire 6 is well inside the blood vessel 5.

The second step of the conventional method involves the removal of the introducer 1, thereby leaving only the guide wire 6 in place, as is illustrated in Fig. 3. Here, the basic disadvantage of the conventional method is apparent. The diameter of the guide wire 6 is much less than the diameter of the puncture hole, and in particular less than the diameter of the hole in the wall 4 of the blood vessel 5, which means that blood can flow out from the blood vessel 5. At this stage, external manual compression pressure is usually applied (not shown) in order to reduce the flow of blood out from the blood vessel 5. Besides the problem that it can be difficult to apply enough compression pressure to completely prevent bleeding from the blood vessel 5, some blood will always flow out from the blood vessel 5. The reason for this is that the compression pressure cannot be applied during the insertion of a second introducer, since compression of the tissue 3 and the vessel 4 would apparently prevent this insertion.

Fig. 4 shows the third step of the conventional method, in which a second introducer 7 and a dilator 8 are passed over the guide wire 6 until the distal end of the introducer 7 is inside the blood vessel 5. In subsequent steps (not shown), first the guide wire 6 and then the dilator 8 are removed, thereby leaving only the second introducer 7 in place, as is illustrated in Fig. 5. Here it should be noted that to accomplish the substitution illustrated in Fig. 1 to Fig. 5, both the guide wire 6 as well as the dilator 8 are needed. It can also be mentioned that the removal of the introducer 1, the application of the manual compression and the insertion of the second introducer 7, with the dilator 8 inserted therein, sometimes can be accomplished by a single skilful doctor, but often a second medically trained person is required.

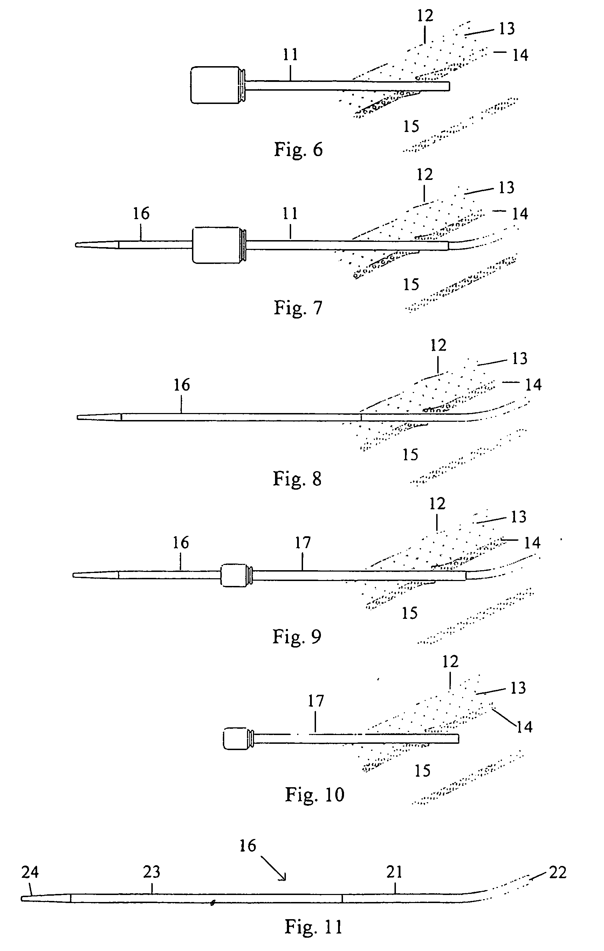

The replacement method which can be carried out with the system according to the invention is now going to be described. Like the conventional method described in conjunction with Fig. 1 to Fig. 5, the present replacement method could be used whenever one wants to replace a first introducer with a second introducer. The starting point of the present method is the same as for the conventional method, and in Fig. 6 is illustrated a situation in which a first introducer 11 has been inserted through a puncture hole, which extends from the skin 12 of a patient, through tissue 13 and through the wall 14 of a blood vessel 15, such that the distal end of the introducer 11 is inside the blood vessel 15 while the proximal end of the introducer 11 is outside the skin 12.

In Fig. 7, the first step of the present method is shown. In this first step, a guide rod 16, extended in length between a proximal end and a distal end, is introduced into the introducer 11 until the distal end of the guide rod 16 is well inside the blood vessel 15.

The second step of the present method involves the removal of the introducer 11, thereby leaving only the guide rod 16 in place, as is illustrated in Fig. 8. Here, one advantage of the present method is apparent. Transversely to its length, the guide rod 16 has a sectional dimension or diameter adapted to receive the new introducer in close relation about the circumference of the rod 16. The diameter of the guide rod 16 is equal, or almost equal, to the inner diameter of the introducer 11 in order to guide the introducer in sliding movement from the proximal end towards the distal end of the rod 16. Also, the sectional dimension or diameter of a distal portion of the guide rod 16 is equal, or almost equal, to the diameter of the puncture hole in the wall 14 of the blood vessel 15. Furthermore, when the introducer 11 is removed, the hole in the vessel wall 14 contracts slightly around the distal portion of the guide rod 16. This means that the vessel wall around the puncture hole will be supported by the distal portion of the rod 16 when the distal end of the rod remains inside the vessel for insertion of the new introducer, and there will be essentially no flow of blood out from the blood vessel 15. This is an advantage in itself; and since no external manual compression pressure has to be applied, one manual operation less has to be executed. One operation less may sound insignificant, but could actually imply that one medically trained person is saved for other duties, or if only one doctor is carrying out the operation, that he or she can concentrate on the insertion of the second introducer.

Fig. 9 shows the third step of the present method, in which a second introducer 17 is passed over the guide rod 16 until the distal end of the introducer 17 is inside the blood vessel 15. In a subsequent step (not shown), the guide rod 16 is removed, thereby leaving only the second introducer 17 in place, as is illustrated in Fig. 10. Here it should be noted that to accomplish the substitution illustrated in Fig. 6 to Fig. 10, only one instrument, i.e. the guide rod 16, is needed, which is in contrast to the conventional method in which two instruments, i.e. the guide wire and the dilator, were needed.

The guide rod 16 used in the present method described above is illustrated separately in Fig. 11. The guide rod 16 has a distal portion 21 with a distal end 22 and a proximal portion 23 with a proximal end 24. The distal portion 21 should preferably be flexible, so that the risk of damaging a vessel during insertion is minimized. To further reduce this risk, the distal end 22 should preferably be blunt. Further, the distal portion 21 should preferably be made from a material that can be pre-bent in such a way that the distal portion 21, after its passage through an introducer, resumes its bent shape. The bent shape, which also is common for a conventional guide wire, facilitates the insertion of the guide rod 16 into a vessel. To facilitate the threading of an introducer over the guide rod 16, the proximal portion 23 should preferably be stiff and the proximal end 24 of the proximal portion 23 should preferably be tapered. With a stiff proximal portion 23 having a tapered end 24 it is considerably easier to thread an introducer over the guide rod 16, than to thread a dilator over a guide wire, as is done in the conventional method. The guide rod 16 could be made from any suitable materials, such as plastic or metal, and the distal portion could, for example, be made in the form of a coil spring. In the Fig. 11 embodiment, the guide rod 16 has an outer diameter of 3 French (1 mm) or greater, such as 6 French, 7 French, 8 French, or more.

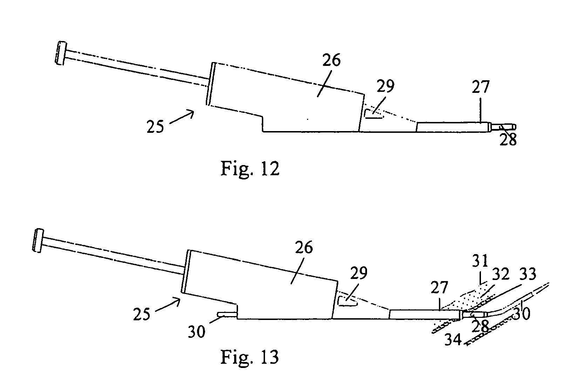

Above, the second introducer, such as the introducer 17 illustrated in Fig. 10, has been described as a separate item, to which a tool for sealing a percutaneous puncture may be connected, as, for example, is described in the above-mentioned US 6,090,130. It should, however, be understood that the system according to the present invention equally well can be used in a situation where the second introducer is an integrated part of a tool for closing a puncture wound. The term "introducer" is therefore herein meant to encompass both separate introducers and introducers which are part of another device. In Fig. 12 such a tool 25 for closing a puncture wound is illustrated. The tool 25 comprises basically a housing 26 and a distal introducer 27, which is fixedly connected to the housing 26. The introducer 27 is provided with a first hole 28, which through a canal (not shown in the figure) is in fluid communication with an opening 29 provided in the housing 26. This canal can be provided as a longitudinal recess in the surface of a guide rod over which the introducer 27 is threaded, or the canal can be provided as a longitudinal, internal hole within the guide rod.

In Fig. 13, the introducer 27 of the tool 25 has, over a guide rod 16, been inserted from the skin 31 of a patient, through tissue 32 and through the wall 33 of a blood vessel 34. The introducer 27 is positioned by means of the same positioning operation as was illustrated in Fig. 7 to Fig. 9, and the introducer 27 of Fig. 13 is therefore in the same position as the introducer 17 of Fig. 9, with the difference that the introducer 27 is a part of a tool 25 for closing a puncture wound rather than being a separate introducer. In the position shown in Fig. 13, blood can flow into the hole 28 in the introducer 27 through a canal provided in the guide rod 16 and out from the opening 29 provided in the housing 26, thereby providing a user with a verification that the introducer 27 is at the correct position with the vessel 34.

Although the present invention has been described with reference to specific embodiments, also shown in the appended drawings, it will be apparent for those skilled in the art that many variations and modifications can be done within the scope of the invention as described in the specification and defined with reference to the following claims. It should, for example, be clear that the system according to the present invention is not limited to be used in blood vessels, but could be used in any vessel when a first introducer has to be replaced with a second introducer. As another example, the guide rod could be provided with a measurement scale, for example, in the form of centimeter or millimeter marks, that provide a user with an indication of how far the guide rod is inserted into the introducer.

## Claims

1. A system comprising a first introducer (11), a second introducer (17), and a guide which is operated for insertion of the second introducer in replacement of the first introducer such that a distal end of said second introducer reaches through a puncture hole in a vessel wall to be located inside the vessel, while a proximal end of the second introducer being located outside the skin of a patient, the guide comprising:
- a guide rod (16) having a length between a proximal end (24) and a distal end (22), the guide rod being insertable through the first introducer until the distal end of the guide rod is located in the vessel, and after withdrawal of the first introducer in proximal direction, effective for guiding the second introducer in sliding movement from the proximal end towards the distal end of the guide rod until the distal end of the second introducer is located in the vessel;
- the guide rod (16) having a proximal portion (23) adjoining a distal portion (21), **characterized in that** an outer diameter of said proximal and distal portions being equal, or almost equal to the inner diameter of the first introducer and effective for guiding the first and the second introducers in close relation about the circumference of the guide rod, whereby the diameter of the distal portion (21) of the guide rod is dimensioned for supporting the vessel wall around the puncture hole when the guide rod alone is located with its distal end inside the vessel after withdrawal of the first introducer (11).

2. The system of claim 1, wherein the distal portion (21) of the rod is flexible.

3. The system of claim 1 or 2, wherein the proximal portion (23) of the rod is not flexible.

4. The system of any previous claim, wherein the distal portion (21) has a blunt end.

5. The system of any previous claim, wherein the proximal portion (23) has a tapered end.

6. The system of any previous claim, wherein the distal portion (21) can be pre-bent in such a way that it resumes its bent shape after having passed through the first introducer.

7. The system of any previous claim, wherein a diameter of the guide rod is equal, or almost equal, to an inner diameter of the first and second introducers.

8. The system of any previous claim, further comprising a measurement scale.

9. The system of any previous claim, wherein the surface of the guide rod is provided with a longitudinal recess.

10. The system of any previous claim, wherein the guide rod is provided with a longitudinal, internal hole.

## Patentansprüche

1. System umfassend eine erste Einführvorrichtung (11), eine zweite Einführvorrichtung (17) und eine Führung, die zum Einsetzen der zweiten Einführungsvorrichtung als Ersatz für die erste Einsetzungsvorrichtung so betätigt wird, dass sich ein distales Ende der zweiten Einführungsvorrichtung durch eine Einstichöffnung in einer Gefäßwand so hindurch erstreckt, dass es innerhalb des Gefäßes angeordnet ist, während ein proximales Ende der zweiten Einführvorrichtung außerhalb der Haut eines Patienten angeordnet ist, wobei die Führung umfasst:
- einen Führungsstab (16), der eine Länge zwischen einem proximalen Ende (24) und einem distalen Ende (22) aufweist, wobei der Führungsstab durch die erste Einführungsvorrichtung hindurch einsetzbar ist, bis das distale Ende des Führungsstabes in dem Gefäß angeordnet ist, und nach dem Entnehmen der ersten Einführungsvorrichtung in der proximalen Richtung zur Führung der zweiten Einführvorrichtung in gleitender Bewegung von dem proximalen Ende in Richtung des distalen Endes des Führungsstabes dient, bis das distale Ende der zweiten Einführvorrichtung in dem Gefäß angeordnet ist;
- wobei der Führungsstab (16) einen proximalen Abschnitt (23) aufweist, der an einen distalen Abschnitt (21) angrenzt,
**dadurch gekennzeichnet, dass**
ein äußerer Durchmesser der proximalen und distalen Abschnitte gleich oder nahezu gleich dem inneren Durchmesser der ersten Einführvorrichtung ist und zur Führung der ersten und zweiten Einführungsvorrichtungen in enger Verbindung zu dem Umfang des Führungsstabes dient, wobei der Durchmesser des distalen Abschnitts (21) des Führungsstabes so dimensioniert ist, dass er die Gefäßwand um die Einstichöffnung herum stützt, wenn der Führungsstab alleine nach dem Entnehmen der ersten Einführvorrichtung (11) mit seinem distalen Ende innerhalb des Gefäßes angeordnet ist.

2. System gemäß Anspruch 1, wobei der distale Abschnitt (21) des Stabes flexibel ist.

3. System gemäß Anspruch 1 oder 2, wobei der proximale Abschnitt (23) des Stabes nicht flexibel ist.

4. System gemäß einem der vorstehenden Ansprüche, wobei der distale Abschnitt (21) ein stumpfes Ende aufweist.

5. System gemäß einem der vorstehenden Ansprüche, wobei der proximale Abschnitt (23) ein spitz zulaufendes Ende aufweist.

6. System gemäß einem der vorstehenden Ansprüche, wobei der distale Abschnitt (21) auf eine solche Weise vorgebogen sein kann, dass er seine gebogene Form wieder einnimmt, nachdem er durch die erste Einführvorrichtung hindurch getreten ist.

7. System gemäß einem der vorstehenden Ansprüche, wobei ein Durchmesser des Führungsstabes gleich oder nahezu gleich zu einem inneren Durchmesser der ersten und zweiten Einführvorrichtung ist.

8. System gemäß einem der vorstehenden Ansprüche, weiterhin umfassend eine Messskala.

9. System gemäß einem der vorstehenden Ansprüche, wobei die Oberfläche des Führungsstabes mit einer Längsausnehmung versehen ist.

10. System gemäß einem der vorstehenden Ansprüche, wobei der Führungsstab mit einem sich längs erstreckenden, inneren Hohlraum versehen ist.

## Revendications

1. Système comprenant un premier introducteur (11), un deuxième introducteur (17) et un guide qui est actionné pour réaliser l'insertion du deuxième introducteur en remplacement du premier introducteur, de sorte qu'une extrémité distale dudit deuxième introducteur atteigne un trou de perforation dans une paroi d'un vaisseau devant être située à l'intérieur du vaisseau, alors qu'une extrémité proximale du deuxième introducteur est située à l'extérieur de la peau d'un patient, le guide comprenant :
- une tige de guidage (16) présentant une longueur comprise entre une extrémité proximale (24) et une extrémité distale (22), la tige de guidage pouvant être insérée à travers le premier introducteur jusqu'à ce que l'extrémité distale de la tige de guidage soit située dans le vaisseau et, après le retrait du premier introducteur dans la direction proximale, soit efficace pour guider le deuxième introducteur dans un mouvement coulissant à partir de l'extrémité proximale en direction de l'extrémité distale de la tige de guidage jusqu'à ce que l'extrémité distale du deuxième introducteur soit située dans le vaisseau ;
- la tige de guidage (16) présentant une partie proximale (23) contiguë à une partie distale (21),
**caractérisé en ce qu'**un diamètre extérieur desdites parties proximale et distale est égal ou pratiquement égal au diamètre intérieur du premier introducteur et est efficace pour guider les premier et deuxième introducteurs dans une relation étroite autour de la circonférence de la tige de guidage, le diamètre de la partie distale (21) de la tige de guidage étant dimensionné pour supporter la paroi du vaisseau autour du trou de perforation lorsque la tige de guidage seule est située avec son extrémité distale à l'intérieur du vaisseau après le retrait du premier introducteur (11).

2. Système selon la revendication 1, dans lequel la partie distale (21) de la tige est flexible.

3. Système selon la revendication 1 ou 2, dans lequel la partie proximale (23) de la tige n'est pas flexible.

4. Système selon l'une quelconque des revendications précédentes, dans lequel la partie distale (21) présente une extrémité émoussée.

5. Système selon l'une quelconque des revendications précédentes, dans lequel la partie proximale (23) présente une extrémité conique.

6. Système selon l'une quelconque des revendications précédentes, dans lequel la partie distale (21) peut être prépliée de sorte qu'elle reprenne sa forme pliée après être passée à travers le premier introducteur.

7. Système selon l'une quelconque des revendications précédentes, dans lequel un diamètre de la tige de guidage est égal ou pratiquement égal à un diamètre intérieur des premiers et deuxième introducteurs.

8. Système selon l'une quelconque des revendications précédentes, comprenant en outre une échelle de mesure.

9. Système selon l'une quelconque des revendications précédentes, dans lequel la surface de la tige de guidage comporte un évidement longitudinal.

10. Système selon l'une quelconque des revendications précédentes, dans lequel la tige de guidage comporte un trou interne longitudinal.
